# EUROPÄISCHE PATENTANMELDUNG

(11) **EP 4 616 935 A1**
(43) Veröffentlichungstag der Anmeldung: **17.09.2025**
(21) Anmeldenummer: 24163703.2
(22) Anmeldetag: 15.03.2024
(51) Int. Cl.: B01F 31/441, B01F 33/501, B01F 35/71, B01F 101/20

(54) **KNOCHENZEMENT-MISCHSYSTEM MIT EINSTICHVERBINDUNG**

(71) Anmelder: Heraeus Medical GmbH, 61273 Wehrheim (DE)
(72) Erfinder: Dr. Vogt, Sebastian, 61273 Wehrheim (DE); Dr. Kluge, Thomas, 61273 Wehrheim (DE)
(74) Vertreter: Heraeus IP

(57) **Zusammenfassung**

Die vorliegende Erfindung betrifft eine Vorrichtung zur Herstellung von Knochenzement, aufweisend
ein Basisteil (100), welches einen Behälter (101) zur Aufnahme einer Monomerflüssigkeit (102), ein Bodenteil (103) mit einem Sammelbereich (104), und einen Hohldorn (105) mit einer Spitze (106) aufweist,
und eine Kartusche (200) zur Aufnahme eines PMMA-Knochenzementpulvers, wobei die Kartusche ein Septum (202) aufweist, welches die Kartusche nach außen begrenzt,
wobei die Vorrichtung ausgebildet und eingerichtet ist, mittels Durchstechen des Septums mit dem Hohldorn eine fluidleitende Verbindung zwischen dem Basisteil und der Kartusche zu bilden, um eine Monomerflüssigkeit aus dem Sammelbereich in die Kartusche zu überführen.

## Beschreibung

### GEBIET DER ERFINDUNG

Die vorliegende Erfindung betrifft das Gebiet der Medizintechnik, insbesondere Vorrichtungen zur Herstellung eines PMMA-Knochenzements und zur Lagerung von Komponenten davon.

### STAND DER TECHNIK

Polymethylmethacrylat-Knochenzemente gehen auf die grundlegenden Arbeiten von CHARNLEY zurück. Polymethylmethacrylat-Knochenzemente bestehen üblicherweise aus einer flüssigen Monomerkomponente und einer Pulverkomponente. Die Monomerkomponente kann beispielsweise das Monomer Methylmethacrylat und einen darin gelösten Aktivator (z.B. N,N-Dimethyl-p-toluidin) enthalten. Die Pulverkomponente, auch als Knochenzementpulver bezeichnet, kann ein oder mehrere Polymere aufweisen, die auf Basis von Methylmethacrylat und Comonomeren, wie Styren, Methylacrylat oder ähnlichen Monomeren durch Polymerisation, vorzugsweise Suspensionspolymerisation, herstellbar sein können. Die Pulverkomponente kann weiterhin einen Röntgenopaker und einen Initiator wie z.B. Dibenzoylperoxid aufweisen. Beim Vermischen der Pulverkomponente mit der Monomerkomponente kann durch Quellung der Polymere der Pulverkomponente im Methylmethacrylat ein plastisch verformbarer Teig entstehen, der eigentliche Knochenzementteig. Beim Vermischen der Pulverkomponente mit der Monomerkomponente reagiert beispielsweise der Aktivator N,N-Dimethyl-p-toluidin mit Dibenzoylperoxid unter Bildung von Radikalen. Die gebildeten Radikale können die radikalische Polymerisation des Methylmethacrylates initiieren. Mit fortschreitender Polymerisation des Methylmethacrylates erhöht sich die Viskosität des Zementteigs, bis dieser erstarrt. Polymethylmethacrylat-Knochenzemente können in geeigneten Mischbechern mit Hilfe von Spateln durch Vermischen der Pulverkomponente mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US 6,033,105 A, US 5,624,184 A, US 4,671,263 A, US 4,973,168 A, US 5,100,241 A, WO 99/67015 A1, EP 1 020 167 A2, US 5,586,821 A, EP 1 016 452 A2, DE 36 40 279 A1, WO 94/26403 A1, EP 1 005 901 A2, US 5,344,232 A.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche geschlossenen "Full-Prepacked-Mischsysteme" sind in den Patentdokumenten EP0380867B1, EP0796653B1, EP0692229B1, US5997544A, US6709149B1, DE69812726T2 und US5588745A, WO9416951A1, DE19718648A1, EP1741413B1, EP3054880B1, DE102009031178B3, US8662736B2, EP2269718B1, EP2281532B1 und EP3093067B1 beschrieben.

Bei Full-Prepacked-Mischsystemen stellen sich einige Herausforderungen. Wünschenswert ist der sichere, reproduzierbare Transfer der Monomerflüssigkeit in das Polymethylmethacrylat-Knochenzementpulver und die Vermeidung einer unkontrollierten Mischung der Komponenten während der Lagerung. Vorteilhaft ist, wenn das Zementpulver während des Transports und der Lagerung des Mischsystems nicht in das Leitungsmittel zum Transfer der Monomerflüssigkeit eindringen kann. Ansonsten kann das Leitungsmittel beim Monomertransfer durch Quellung und Verklebung verstopfen. Eine Blockierung des Leitungsmittels durch gequollenen, klebenden Knochenzement könnte den Monomerflüssigkeitstransfer unterbrechen und die Vermischung der Zementkomponenten im vorbestimmten Mischungsverhältnis verhindern.

Bei den Full-Prepacked-Mischsystemen gemäß EP0796653B1, US6709149B1, EP1741413B1, EP3054880B1, EP2269718B1 und EP2281532B1 erfolgt der Monomertransfer durch Anlegen eines externen Vakuums an die Kartusche über einen Vakuumanschluss und einer porösen Platte. Das bedeutet, dass die Monomerflüssigkeit durch das angelegte Vakuum in die Kartusche gesaugt wird. Bei dem in der EP3093067B1 beschriebenen Mischsystem kann entweder ein Transfer der Monomerflüssigkeit in die Kartusche durch Vakuumeinwirkung, oder alternativ ein Drucktransfer der Monomerflüssigkeit durch einen in das Mischsystem integrierten, manuell bedienbaren Pumpkolben erfolgen.

Die Blockierung des Leitungsmittels zum Transfer der Monomerflüssigkeit wird gemäß EP2281532B1 dadurch vermieden, dass ein für die Monomerflüssigkeit permeables Netz oder eine Porenscheibe zum Blockieren der Zementpulverpartikel genutzt wird, wobei das Netz oder die Porenscheibe Bestandteil einer speziellen Düse sind, die einen scharfen Monomerflüssigkeitsstrahl beim Transfer erzeugt, der gequollene Zementpartikel verdrängt, so dass ein vollständiger Monomerflüssigkeitstransfer in das Knochenzementpulver möglich ist. EP1741413B1 offenbart ein Verfahren zum Inkontaktbringen eines Pulvers und einer flüssigen Komponente, wobei ein Flüssigkeitsbehälter durch Eindringen einer Kanüle in einen Wandabschnitt des Flüssigkeitsbehälters geöffnet wird, der ein seitliches Loch in einem inneren Rohrelement im Flüssigkeitsbehälter verschließt.

In der Patentschrift EP3636338B1 ist ein Mischsystem offenbart, das einen Verbindungszylinder aufweist, der dazu konfiguriert ist, selektiv an eine Mischkammer gekoppelt und von dieser entfernbar zu sein, und wobei ein Ventil dazu konfiguriert ist, die Verbindung zwischen dem Verbindungszylinder und dem Ventil abzudichten, wenn sie selektiv miteinander gekoppelt sind, und dazu konfiguriert ist, die Mischkammer abzudichten, wenn der Verbindungszylinder aus der Mischkammer entnommen wird. Die EP3490700B1 offenbart ein ähnliches Mischsystem.

### BEVORZUGTE AUSFÜHRUNGSFORMEN

Eine Aufgabe der vorliegenden Erfindung besteht darin, eines oder mehrere der oben geschilderten und weitere Probleme des Stands der Technik zu lösen. Beispielsweise liefert die Erfindung eine Vorrichtung zum Lagern und Mischen von Knochenzement, die eine einfache und zuverlässige Mischung einer Monomerflüssigkeit mit einer Pulverkomponente eines PMMA-Knochenzements erlaubt, um einen gebrauchsfertigen PMMA-Knochenzementteig daraus herzustellen. Die erfindungsgemäßen Vorrichtungen können mehrere Teile aufweisen, die in einfacher Weise miteinander verbunden und wieder voneinander getrennt werden können, und dabei eine zuverlässige und sichere Überführung einer Monomerflüssigkeit von einem Teil der Vorrichtung in das andere Teil der Vorrichtung erlauben. Ein Austreten von Monomerflüssigkeit oder Eindringen von Kontaminationen können durch ein selbstdichtendes Verbindungssystem vermieden werden. Die erfindungsgemäße Vorrichtung kann eine einfache Handhabung bieten. Eine Monomerflüssigkeit und eine Pulverkomponente eines PMMA-Knochenzements können in der Vorrichtung getrennt voneinander gelagert, kontrolliert zusammengeführt und miteinander gemischt werden. Die Vorrichtung kann diese Funktionen ausüben, während sie sicher auf einer Unterlage aufgestellt ist. Die Monomerflüssigkeit kann vollständig überführt werden, wobei eine Verstopfung der Vorrichtung durch verklumptes Pulver vermieden werden kann.

Diese Aufgaben werden gelöst durch die hierin beschriebenen Verfahren, Vorrichtungen, Kits und medizinischen Verwendungen, insbesondere denjenigen, die in den Patentansprüchen beschrieben sind.

Bevorzugte Ausführungsformen der Erfindung werden nachfolgend beschrieben.

Eine erste Ausführungsform betrifft eine Vorrichtung zur Herstellung von Knochenzement, aufweisend ein Basisteil, welches einen Behälter zur Aufnahme einer Monomerflüssigkeit, ein Bodenteil mit einem Sammelbereich, und einen Hohldorn mit einer Spitze aufweist,
und eine Kartusche zur Aufnahme eines PMMA-Knochenzementpulvers, wobei die Kartusche ein Septum aufweist, welches die Kartusche nach außen begrenzt, wobei die Vorrichtung ausgebildet und eingerichtet ist, mittels Durchstechen des Septums mit dem Hohldorn eine fluidleitende Verbindung zwischen dem Basisteil und der Kartusche zu bilden, um eine Monomerflüssigkeit aus dem Sammelbereich in die Kartusche zu überführen.

Eine zweite Ausführungsform betrifft eine Vorrichtung gemäß der ersten Ausführungsform, wobei das Basisteil weiterhin einen Kolben aufweist, wobei der Kolben eine Durchführung zur Aufnahme des Hohldorns aufweist, wobei der Kolben eingerichtet ist, in einer Grundkonfiguration der Vorrichtung die Spitze des Hohldorns in der Durchführung aufzunehmen, und die Spitze des Hohldorns aus der Durchführung freizugeben, wenn die Kartusche auf den Kolben gedrückt wird.

Eine dritte Ausführungsform betrifft eine Vorrichtung gemäß der zweiten Ausführungsform, wobei das Basisteil eine Feder aufweist, welche zur beweglichen Lagerung des Kolbens innerhalb des Basisteils angeordnet und eingerichtet ist.

Eine vierte Ausführungsform betrifft eine Vorrichtung gemäß einer der zweiten oder dritten Ausführungsform, wobei das Basisteil einen Anschlag aufweist, um die Bewegung des Kolbens und/oder der Kartusche in dem Basisteil zu begrenzen.

Eine fünfte Ausführungsform betrifft eine Vorrichtung gemäß einer der vorangehenden Ausführungsformen, wobei die Vorrichtung einen Filter aufweist, wobei der Filter ausgebildet und eingerichtet ist, das Eindringen von Partikeln aus dem Basisteil in die Kartusche zu verhindern.

Eine sechste Ausführungsform betrifft eine Vorrichtung gemäß einer der vorangehenden Ausführungsformen, wobei die Kartusche einen Anschluss zur Verbindung mit einer Vakuumquelle aufweist, um das Überführen einer Monomerflüssigkeit aus dem Sammelbereich in die Kartusche mithilfe eines Druckunterschieds zu ermöglichen.

Eine siebte Ausführungsform betrifft eine Vorrichtung gemäß einer der vorangehenden Ausführungsformen, wobei der Behälter einen Träger zur Aufnahme einer Glasampulle oder eines Folienbeutels mit Monomerflüssigkeit aufweist.

Eine achte Ausführungsform betrifft eine Vorrichtung gemäß der siebten Ausführungsform, wobei der Behälter einen Ampullenbrecher aufweist, wobei der Ampullenbrecher zum Öffnen einer Glasampulle innerhalb des Behälters eingerichtet ist.

Eine neunte Ausführungsform betrifft eine Vorrichtung gemäß einer der vorangehenden Ausführungsformen, wobei der Ampullenbrecher ausgestaltet und eingerichtet ist, durch Biegen des Behälters relativ zu dem Bodenteil, oder durch Verschieben eines Trägers gegen einen Vorsprung, eine Glasampulle innerhalb des Behälters zu öffnen.

Eine zehnte Ausführungsform betrifft eine Vorrichtung gemäß einer der vorangehenden Ausführungsformen, wobei das Basisteil eingerichtet ist, das Überführen einer Monomerflüssigkeit aus dem Behälter in den Sammelbereich durch freien Gravitationsfluss zu ermöglichen.

Eine elfte Ausführungsform betrifft eine Vorrichtung gemäß einer der vorangehenden Ausführungsformen, wobei der Behälter in einem Winkel zu dem Bodenteil angeordnet ist, welcher 20° bis 80°, bevorzugt 21° bis 55°, beträgt.

Eine zwölfte Ausführungsform betrifft eine Vorrichtung gemäß einer der vorangehenden Ausführungsformen, wobei die Kartusche lösbar mit dem Basisteil verbindbar ist.

Eine dreizehnte Ausführungsform betrifft eine Vorrichtung gemäß einer vorangehenden Ausführungsformen, wobei das Basisteil ein Leitungselement aufweist, wobei das Leitungselement eingerichtet ist, eine fluidleitende Verbindung zwischen dem Sammelbereich und dem Hohldorn herzustellen.

Eine vierzehnte Ausführungsform betrifft eine Vorrichtung gemäß einer der vorangehenden Ausführungsformen, wobei das Basisteil ein Leitungselement aufweist, wobei das Leitungselement eingerichtet ist, eine fluidleitende Verbindung zwischen dem Sammelbereich und dem Hohldorn herzustellen.

Eine fünfzehnte Ausführungsform betrifft eine Vorrichtung gemäß einer der vorangehenden Ausführungsformen, wobei der Sammelbereich eine Vertiefung aufweist, und wobei der Hohldorn bevorzugt fluidleitend mit dem tiefsten Punkt der Vertiefung verbunden ist.

Eine sechzehnte Ausführungsform betrifft eine Vorrichtung gemäß einer der vorangehenden Ausführungsformen, wobei die Vorrichtung ausgestaltet und eingerichtet ist, eine Monomerflüssigkeit aus dem Basisteil in die Kartusche zu überführen, während das Basisteil auf einer Unterlage aufgestellt ist, so dass das Bodenteil in Richtung der Unterlage ausgerichtet ist.

### KURZE BESCHREIBUNG DER ZEICHNUNGEN

**Figur 1** zeigt den Querschnitt eines Basisteil einer erfindungsgemäßen Vorrichtung.
**Figur 2** zeigt den Querschnitt einer Kartusche einer erfindungsgemäßen Vorrichtung.
**Figur 3** zeigt das Aufbrechen einer Glasampulle in einem Basisteil.
**Figur 4** zeigt das Überführen von Monomerflüssigkeit aus einem Basisteil in eine Kartusche.
**Figur 5** zeigt ein Basisteil mit einem Ampullenbrecher, der einen verschiebbaren Träger und einen Vorsprung aufweist.
**Figur 6** zeigt ein Basisteil gemäß Figur 5, in welchem Monomerflüssigkeit aus aufgebrochenen Ampullen in den Sammelbereich überführt ist.
**Figur 7** zeigt ein Basisteil, das mit einer Kartusche verbunden ist.
**Figur 8** zeigt eine Kartusche während der Abgabe eines Knochenzementteigs.
**Figur 9** zeigt eine isometrische Ansicht einer erfindungsgemäßen Vorrichtung, wobei das Basisteil von der Kartusche getrennt ist.

### AUSFÜHRLICHE BESCHREIBUNG

Zu den hierin beschriebenen Ausführungsformen, deren Elemente ein bestimmtes Merkmal (z.B. ein Material) "aufweisen", "enthalten", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "enthalten", "enthaltend", "aufweisen" oder "aufweisend" verwendet.

Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist.

Soweit nicht anders angegeben oder aus dem Zusammenhang eindeutig ausgeschlossen, ist es grundsätzlich möglich und wird hiermit eindeutig in Betracht gezogen, dass Merkmale unterschiedlicher Ausführungsformen auch in den anderen hierin beschriebenen Ausführungsformen vorhanden sein können. Ebenso wird grundsätzlich erwogen, dass alle Merkmale, die hierin in Zusammenhang mit einem Verfahren beschrieben werden, auch für die hierin beschriebenen Erzeugnisse, Vorrichtungen, Kits und Verwendungen anwendbar sind, und umgekehrt. Lediglich aus Gründen der knapperen Darstellung werden alle diese erwogenen Kombinationen nicht in allen Fällen explizit aufgeführt. Auch technische Lösungen, die zu den hierin beschriebenen Merkmalen bekanntermaßen gleichwertig sind, sollen grundsätzlich vom Umfang der Erfindung umfasst sein.

Eine erste Ausführungsform betrifft eine Vorrichtung zur Herstellung von Knochenzement, aufweisend ein Basisteil, welches einen Behälter zur Aufnahme einer Monomerflüssigkeit, ein Bodenteil mit einem Sammelbereich, und einen Hohldorn mit einer Spitze aufweist,
und eine Kartusche zur Aufnahme eines PMMA-Knochenzementpulvers, wobei die Kartusche ein Septum aufweist, welches die Kartusche nach außen begrenzt, wobei die Vorrichtung ausgebildet und eingerichtet ist, mittels Durchstechen des Septums mit dem Hohldorn eine fluidleitende Verbindung zwischen dem Basisteil und der Kartusche zu bilden, um eine Monomerflüssigkeit aus dem Sammelbereich in die Kartusche zu überführen.

Die erfindungsgemäße Vorrichtung kann zwei miteinander verbindbare Teile aufweisen, nämlich ein Basisteil und eine Kartusche. Das Basisteil weist einen Behälter zur Aufnahme einer Monomerflüssigkeit auf. Der Behälter kann einen Träger zur Aufnahme einer Glasampulle oder eines Beutels aufweisen. Solche Glasampullen oder Beutel werden als handelsübliche Verpackungen für Monomerflüssigkeit eines PMMA-Knochenzements verwendet. Der Behälter kann mit Monomerflüssigkeit, zum Beispiel gasdicht verpackt in einer solchen Glasampulle oder einem Beutel, vorbefüllt sein. Alternativ kann der Behälter leer bereitgestellt und vom Benutzer mit Monomerflüssigkeit befüllbar sein, beispielsweise durch Einbringen einer Glasampulle mit Monomerflüssigkeit in den Behälter. Ebenso kann die Kartusche leer oder mit PMMA-Knochenzementpulver vorbefüllt bereitgestellt werden. Der Behälter kann einen transparenten Bereich aufweisen. Der Sammelbereich kann ebenfalls einen transparenten Bereich aufweisen. Dies kann es einem Benutzer der Vorrichtung erlauben, das vollständige Abfließen von Monomerflüssigkeit aus dem Behälter in den Sammelbereich von außen visuell zu überprüfen. Weiterhin kann das Basisteil eine Füllstandsmarkierung aufweisen.

Das Basisteil kann zum Aufstellen auf eine Unterlage eingerichtet sein. Das Basisteil weist ein Bodenteil auf, das eine flache Außenseite aufweisen kann. Das Bodenteil weist einen Sammelbereich auf. Der Sammelbereich ist bevorzugt zur Aufnahme von Monomerflüssigkeit ausgebildet und eingerichtet. Beispielsweise kann das Basisteil eingerichtet sein, Monomerflüssigkeit aus einer Verpackung, wie z.B. einer Glasampulle oder einem Folienbeutel, freizusetzen, und durch Gravitationsfluss in den Sammelbereich zu überführen. Der Sammelbereich kann eine Vertiefung aufweisen, beispielsweise eine konkave Wölbung, vergleichbar mit einer Uhrglasschale. Der Querschnitt der Vertiefung kann U-förmig oder V-förmig sein. Die Vertiefung kann eine trichterförmige Form aufweisen.

Der Sammelbereich weist bevorzugt eine Aufnahmekapazität für Monomerflüssigkeit auf, welche das Volumen der in dem Behälter enthaltenen oder aufnehmbaren Monomerflüssigkeit übersteigt. Beispielsweise kann der Sammelbereich ein Volumen aufweisen, welches mindestens das 1,1-fache Volumen der in dem Behälter enthaltenen oder aufnehmbaren Monomerflüssigkeit beträgt. Wenn beispielsweise der Behälter einen Träger zur Aufnahme von handelsüblichen Glasampullen mit 10 ml Monomerflüssigkeit aufweist, weist der Sammelbereich bevorzugt ein Volumen von mindestens 11 ml auf. Dies gilt in entsprechender Weise für Ausführungsformen, die einen Träger zur Aufnahme von Glasampullen mit 5 ml, 20 ml, oder 30 ml Monomerflüssigkeit aufweisen.

Das Basisteil weist weiterhin einen Hohldorn mit einer Spitze auf. Der Hohldorn weist bevorzugt einen Innenraum auf, der das Hindurchfließen einer Flüssigkeit durch den Hohldorn erlaubt. Der Innenraum kann einen Durchmesser von 1,0 mm bis 3,0 mm aufweisen. Der Hohldorn kann Auslassöffnungen mit einen Durchmesser kleiner 1,5 mm und besonders bevorzugt kleiner 1,0 mm aufweisen. Durch einen geringen Durchmesser des Hohldorns kann beim Überführen einer Monomerflüssigkeit aus dem Sammelbereich in die Kartusche ein scharfer Strahl ausgebildet werden, welcher gegebenenfalls kleinere Partikel aus Knochenzementpulver verdrängen kann. Auf diese Weise kann eine Verstopfung des Hohldorns vermieden werden. Der Hohldorn kann eine angeschrägte Spitze, ähnlich einer geschliffenen Kanüle, aufweisen, wobei sich bevorzugt in einer Schräge der Spitze eine Auslassöffnung des Hohldorns befindet. Der Hohldorn kann eine runde Spitze aufweisen. Hierbei können nahe der der Spitze ein oder mehrere Öffnungen angeordnet sein, die mit dem Innenraum des Hohldorns verbunden sind. Weiterhin kann der Innenraum des Hohlraums einen Absatz aufweisen, an dem ein poröser für Flüssigkeiten durchlässiger Stift gestützt ist. Dieser Stift kann als Filter dienen, wie nachfolgend hierin beschrieben. Der Innenraum des Hohldorns kann in Richtung des Absatzes verjüngt sein. Der Absatz kann eingerichtet sein, den Stift in dem Hohldorn zu halten. Der Hohldorn kann aus Kunststoff oder Metall, z.B. Edelstahl, ausgebildet sein. Beispiele für verwendbare Kunststoffe umfassen Polyamid 12, Polyamid 6, Polyamid 66 und Polyethersulfon. Der Hohldorn, insbesondere die Spitze des Hohldorns, kann durch eine abnehmbare Schutzkappe bedeckt sein.

Die Kartusche weist ein Septum auf, welches die Kartusche nach außen begrenzt. Das Septum ist bevorzugt in einem Bereich der Kartusche angeordnet, der zur Verbindung mit dem Basisteil ausgebildet und eingerichtet ist. Das Septum ist mit dem Hohldorn des Basisteils durchstechbar. In einer Konfiguration der Vorrichtung, in welcher das Septum mit dem Hohldorn durchstochen ist, kann sich eine fluidleitende Verbindung zwischen dem Basisteil und der Kartusche bilden. Dies ermöglicht es, eine Monomerflüssigkeit aus dem Sammelbereich des Basisteils in die Kartusche zu überführen. Hierbei kann durch die selbstdichtende Funktion des Septums verhindert werden, dass Monomerflüssigkeit aus der Vorrichtung nach außen dringt. Dies gilt auch, wenn der Hohldorn wieder aus dem Septum herausgezogen wird, da sich die Einstichstelle durch die Rückstellkraft des Septums selbsttätig verschließen kann. Weiterhin kann hierdurch verhindert werden, dass Kontaminationen von außen in die Vorrichtung eindringen.

In einigen Ausführungsformen weist das Septum ein flexibles, selbstdichtendes Polymer auf. Das Polymer kann ein polyhalogeniertes Olefin, ein Silikon, Kautschuk, Butylkautschuk, Ethylen-Propylen-Dien-Kautschuk (EPDM), oder ein ähnliches Elastomer aufweisen. Das Septum ist bevorzugt eingerichtet, nach einem Durchstechen des Septums mit dem Hohldorn die Kartusche flüssigkeitsdicht zu verschließen. Das Septum ist bevorzugt dehnbar und gummielastisch, wie es beispielsweise von Septen in Chemikalienflaschen bekannt ist. Dadurch kann sich das Septum nach einem Durchstich selbst heilen, d.h. selbsttätig abdichten. Bevorzugt weist das Septum ein biokompatibles Material auf. Das Septum kann eine kreisförmige Form aufweisen. In einer Ausführungsform weist das Septum ein Material auf, das mit einer medizinischen Injektionskanüle gemäß der Norm ISO 7864:2016 mit einer Kraft von weniger als 100 N durchstechbar ist. Das Septum ist bevorzugt flüssigkeitsdicht mit der Kartusche verbunden. In einer Ausführungsform ist das Septum ausgebildet und eingerichtet, nach einem Einstich und anschließender Entfernung des Hohldorns eine Einstichstelle in dem Septum flüssigkeitsdicht zu verschließen. Dies kann durch die Rückstellkraft eines gummielastischen Materials des Septums erfolgen.

Die Kartusche kann ein Führungselement aufweisen, welches ein Einführen des Hohldorns in eine gewünschte Position des Septums erleichtert, z.B. eine Einführungsschräge.

Das Septum kann in einer Halterung gelagert sein. Die Halterung kann lösbar mit der Kartusche verbunden sein. Die Halterung des Septums kann als Kolben ausgestaltet sein. Dieser Kolben kann zur Abgabe von Knochenzementteig aus der Kartusche ausgebildet und eingerichtet sein. Die Halterung für das Septum kann in einem Kartuschenkopf angeordnet sein. Der Kartuschenkopf kann als lösbarer Verschluss der Kartusche ausgebildet sein.

Die Kartusche und das Basisteil sind bevorzugt getrennt voneinander lagerbar, d.h. sie sind als separate Teilelemente ausgestaltet, die lösbar miteinander verbindbar sind. Dadurch kann eine Ermüdung der Rückstellkraft des Septums während der Lagerung verhindert und das selbsttätige Schließen des Septums erleichtert werden. Ein unerwünschter Austritt einer Monomerflüssigkeit aus der Kartusche nach dem Entfernen des Hohldorns aus dem Septum kann verhindert werden. Weiterhin hat die getrennte Lagerung den Vorteil, dass Packmittel mit geringen Abmessungen verwendbar sind.

In einer Ausführungsform weist die Vorrichtung weiterhin einen Kolben auf. Der Kolben kann innerhalb des Basisteils angeordnet sein. Der Kolben kann eine Durchführung zur Aufnahme des Hohldorns aufweisen. Die Durchführung kann sich von einem Innenraum des Basisteils nach außen erstrecken. Der Kolben kann eingerichtet sein, in einer Grundkonfiguration der Vorrichtung die Spitze des Hohldorns in der Durchführung aufzunehmen. In einer solchen Grundkonfiguration der Vorrichtung wird keine äußere Kraft auf die Vorrichtung ausgeübt. Die Spitze des Hohldorns ist in diesem Zustand innerhalb des Kolbens angeordnet, so dass ein Benutzer vor Verletzung durch Kontakt mit der Spitze geschützt ist. In einer Ausführungsform ist der Kolben eingerichtet, die Spitze des Hohldorns aus der Durchführung freizugeben, wenn die Kartusche auf den Kolben gedrückt wird. Dies ermöglicht es, mit der Kartusche Druck auf den Kolben auszuüben, um den Hohldorn freizugeben und das Septum der Kartusche zu durchstechen. Hierbei kann die Spitze des Hohldorns aus der Durchführung herausragen. Somit können Kartusche und Basisteil verletzungssicher fluidleitend miteinander verbunden werden, um eine Monomerflüssigkeit in die Kartusche aufzunehmen. Der Kolben ist bevorzugt eingerichtet, durch die Kartusche in das Basisteil eingeschoben zu werden, wenn die Kartusche mit dem Basisteil verbunden ist. Durch Ausübung von Druck gegen den Kolben durch die Kartusche ist der Kolben demnach bewegbar.

In einer Ausführungsform weist das Basisteil eine Feder auf, welche zur beweglichen Lagerung des Kolbens innerhalb des Basisteils angeordnet und eingerichtet ist. Hierdurch kann der Kolben in einer Position gehalten werden, in der die Spitze des Hohldorns wie oben beschrieben in der Durchführung des Kolbens aufgenommen und somit verletzungssicher innerhalb des Kolbens gehalten ist. Die Feder kann eine Spiralfeder sein. In einer Ausführungsform umgibt die Feder den Hohldorn in einem Abschnitt des Hohldorns, der sich außerhalb der Durchführung des Kolbens befindet. Die Feder kann zwischen dem Bodenteil und dem Kolben angeordnet sein.

In einer Ausführungsform, in welcher der Hohldorn in einer zylinderförmigen Halterung gelagert ist, wie hierin beschrieben, kann die Feder zwischen dieser Halterung und dem Kolben angeordnet sein. Die Feder kann die Trennung von Basisteil und Kartusche erleichtern. Weiterhin kann die Spitze des Hohldorns durch die Feder selbsttätig zurück in den Hohlraum bewegt werden, sobald keine externe Kraft mehr auf den Kolben einwirkt, z.B. wenn der Benutzer die Kartusche nicht mehr aktiv gegen das Basisteil drückt.

In einer Ausführungsform weist das Basisteil einen Anschlag auf. Der Anschlag kann ausgebildet und eingerichtet sein, die Bewegung des Kolbens und/oder der Kartusche in dem Basisteil zu begrenzen. Der Anschlag kann eingerichtet sein, den Kolben im Basisteil zu halten. In einer Ausführungsform, in welcher das Basisteil eine Feder aufweist, kann der Anschlag den Verfahrweg des Kolbens in Richtung der Krafteinwirkung der Feder begrenzen. Hierdurch kann verhindert werden, dass der Kolben durch die Feder aus dem Basisteil herausgeschoben wird. In einer Ausführungsform ist der Anschlag eingerichtet, den Kolben in einer Grundkonfiguration der Vorrichtung in einer Position zu halten, in welcher der Kolben gemeinsam mit einer Außenfläche des Basisteils bündig abschließt. In einer Ausführungsform ist der Anschlag eingerichtet, den Kolben in einer Position zu halten, in der die Spitze des Hohldorns in der Durchführung des Kolbens aufgenommen ist. Weiterhin kann der Anschlag ausgebildet und eingerichtet sein, den Verfahrweg der Kartusche in den Innenraum des Basisteils zu begrenzen. Hierdurch kann die Kartusche in einer gewünschten Position auf das Basisteil aufgestellt werden, ohne dass sich Kartusche und Basisteil gegeneinander bewegen. Es kann ein lösbares Verbindungselement vorgesehen sein, um die Kartusche auf dem Basisteil in einer gewünschten Position zu halten. In einer solchen gewünschten Position kann die Kartusche bündig mit dem Anschlag abschließen. Durch dieses Verbindungselement kann vermieden werden, dass die Feder die Kartusche aus dem Basisteil geschoben wird. Somit muss der Benutzer in einer solchen Ausführungsform während der Benutzung der Vorrichtung den Druck der Feder nicht mit eigener Kraft kompensieren, um die Kartusche in Position zu lasten. Die Kartusche kann einen Vorsprung aufweisen, der ausgebildet und eingerichtet ist, in den Anschlag einzugreifen.

In einer Ausführungsform weist die Vorrichtung einen Filter auf. Der Filter kann im Basisteil angeordnet sein. Beispielsweise kann der Filter zwischen dem Behälter und dem Hohldorn angeordnet sein. Der Filter ist bevorzugt ausgebildet und eingerichtet, ein Eindringen von Partikeln aus dem Basisteil in die Kartusche zu verhindern. Beispielsweise kann der Filter angeordnet und eingerichtet sein, Glasbruchstücke von einer in dem Basisteil aufgebrochenen Glasampulle zurückhalten. Der Filter kann beispielsweise ein Metalldrahtgeflecht oder ein Netz aus einem Kunststoffgewebe sein. Der Filter kann auch eine mit zweckmäßig dimensionierten Löchern versehene Scheibe aus Metall oder Kunststoff sein. Weiterhin können offenporige Schaumstoffe wie zum Beispiel Porex verwendet werden. Bevorzugt sollte der Filter aus einem Material gefertigt sein, welches durch die in einer aufnehmbaren Glasampulle befindliche Monomerflüssigkeit nicht angegriffen wird.

In einer Ausführungsform weist die Kartusche einen Anschluss zur Verbindung mit einer Vakuumquelle auf. Ein solcher Anschluss kann das Überführen einer Monomerflüssigkeit aus dem Sammelbereich in die Kartusche mithilfe eines Druckunterschieds ermöglichen. Beispielsweise kann mit Hilfe einer Vakuumpumpe, die an die Kartusche angeschlossen werden kann, ein Unterdruck in der Kartusche erzeugt werden, um Monomerflüssigkeit aus dem Basisteil durch die fluidleitende Verbindung, die durch Durchstechen des Septums mit dem Hohldorn hergestellt werden kann, in die Kartusche aufzunehmen. Der Anschluss ist bevorzugt derart von dem Septum beabstandet, dass keine Monomerflüssigkeit in den Anschluss gelangen kann, insbesondere in einer Konfiguration, in welcher das Basisteil auf einer Unterlage aufgestellt ist. Beispielsweise können Septum und Anschluss an einander gegenüberliegenden Seiten der Kartusche angeordnet sein.

In einer Ausführungsform weist der Behälter einen Ampullenbrecher auf. Der Ampullenbrecher kann zum Öffnen einer Glasampulle innerhalb des Behälters eingerichtet sein. Der Behälter kann auch ein anderes Mittel zum Freisetzen einer Monomerflüssigkeit aus einer Verpackung, z.B. aus einem Folienbeutel, aufweisen. Dies kann beispielsweise ein Dorn sein, der zum Durchstechen eines Folienbeutels geeignet ist.

In einer Ausführungsform ist der Ampullenbrecher ausgestaltet und eingerichtet, durch Biegen des Behälters relativ zu dem Bodenteil eine Glasampulle innerhalb des Behälters zu öffnen. Hierzu kann der Behälter ein flexibles Gehäuseteil aufweisen, das die Ausübung einer Kraft auf eine Glasampulle in dem Behälter ermöglicht. Der Ampullenbrecher kann einen starren Gehäuseteil oder Vorsprung aufweisen, gegen den eine Glasampulle zum Aufbrechen gedrückt werden kann.

In einer Ausführungsform ist das Basisteil eingerichtet, das Überführen einer Monomerflüssigkeit aus dem Behälter in den Sammelbereich durch freien Gravitationsfluss zu ermöglichen. "Freier Gravitationsfluss" bedeutet hierin, dass eine Monomerflüssigkeit allein durch die Wirkung der Schwerkraft aus dem Behälter in den Sammelbereich gelangen kann, ohne dass hierzu beispielsweise ein Druckunterschied zwischen dem Behälter und dem Sammelbereich erforderlich ist. Bevorzugt erlauben die hierin beschriebenen Vorrichtungen eine vollständige Überführung der Monomerflüssigkeit aus dem Behälter in den Sammelbereich durch freien Gravitationsfluss innerhalb von 20 Sekunden. Anschließend kann die Monomerflüssigkeit durch Ansaugen aus dem Sammelbereich in die Kartusche überführt werden, wie hierin beschrieben.

In einer Ausführungsform ist der Behälter in einem Winkel zu dem Bodenteil angeordnet, welcher 20° bis 80°, bevorzugt 21° bis 55°, beträgt. Dieser Winkel ist durch die Innenseite von jeweils Behälter und Bodenteil definiert, wo Behälter und Bodenteil in Richtung des Bodenteils (d.h. in Richtung der "Unterseite" des Basisteils) an einer senkrechten Seitenwand des Bodenteils ineinander übergehen. Dies entspricht der Stelle, an der bei bestimmungsgemäßem Gebrauch der Vorrichtung die Monomerflüssigkeit aus dem Behälter in den Sammelbereich fließt. Der Behälter ist bevorzugt gegenüberliegend zum Bodenteil angeordnet, d.h. bei bestimmungsgemäßem Gebrauch der Vorrichtung "nach oben" ausgerichtet, um ein Abfließen der Monomerflüssigkeit in den Sammelbereich zu ermöglichen. Beispielsweise kann der Behälter zum übrigen Teil des Basisteils in ähnlicher Weise wie ein Flügel einer Flügelmutter angeordnet sein. Das Basisteil kann beispielsweise zwei oder mehr Behälter aufweisen. In einer Ausführungsform umfasst das Basisteil zwei Y-förmig angeordnete Behälter. Der Kolben kann hierbei zwischen den Behältern angeordnet sein, d.h. zwischen den beiden Y-Armen. Der Behälter kann angeordnet sein, so dass sich dieser in einer Konfiguration des Basisteils, in welcher das Basisteil auf einer Unterlage aufgestellt ist, wobei das Bodenteil in Richtung der Unterlage ausgerichtet ist, oberhalb des Sammelbereichs befindet. Dies erlaubt ein zuverlässiges Abfließen von Monomerflüssigkeit aus dem Behälter in den Sammelbereich.

In einer Ausführungsform ist eine Längsachse des Behälters in einem Winkel zu einer Achse, die senkrecht zu einer Aufstandsfläche des Bodenteils verläuft, angeordnet, welcher 20° bis 80°, bevorzugt 21° bis 55°, beträgt. Bei einem Winkel von 21° bis 55° kann die Ausbildung eines Meniskus durch die Monomerflüssigkeit in besonderem Maße verhindert werden.

In einer Ausführungsform ist die Kartusche lösbar mit dem Basisteil verbindbar. Hierzu können an der Kartusche und am Basisteil entsprechende Verbindungselemente vorgesehen sein, wie z.B. ein Gewinde. Die Verbindungselemente können derart angeordnet sein, dass sie eine Verbindung des Hohldorns mit dem Septum erlauben. Weiterhin kann die Vorrichtung ein Führungselement aufweisen, welche die Verbindung des Basisteils mit der Kartusche in einer gewünschten Orientierung erleichtert. Bevorzugt sind Basisteil und Kartusche derart miteinander verbindbar, dass der Kolben und das Septum bündig miteinander abschließen. Hierzu kann beispielsweise das Basisteil einen überstehenden Bereich aufweisen, der ausgestaltet und eingerichtet ist, in die Kartusche einzugreifen.

In einer Ausführungsform weist das Basisteil ein Leitungselement auf, wobei das Leitungselement eingerichtet ist, eine fluidleitende Verbindung zwischen dem Sammelbereich und dem Hohldorn herzustellen. Das Leitungselement ermöglicht eine Überführung von Monomerflüssigkeit aus dem Sammelbereich in den Hohldorn, bevorzugt eine Überführung durch freien Gravitationsfluss. Das Leitungselement kann beispielsweise als Schlauch, Rohr oder Durchführung ausgestaltet sein. In einer Ausführungsform, in welcher der Hohldorn in einer zylinderförmigen Halterung gelagert ist, wie hierin beschrieben, kann das Leitungselement als Durchführung in der Halterung ausgestaltet sein.

In einer Ausführungsform ist die Vorrichtung ausgestaltet und eingerichtet, eine Monomerflüssigkeit aus dem Basisteil in die Kartusche zu überführen, während das Basisteil auf einer Unterlage aufgestellt ist, so dass das Bodenteil in Richtung der Unterlage ausgerichtet ist. Hierbei weisen die Behälter und die Kartusche bevorzugt nach "oben", d.h. in entgegengesetzter Richtung zur Unterlage, und entgegen der Schwerkraftrichtung. Die Monomerflüssigkeit kann daher durch die Wirkung der Schwerkraft aus dem Behälter in den Sammelbereich fließen.

In einigen Ausführungsformen weist die Kartusche ein PMMA-Knochenzementpulver auf. Das PMMA-Knochenzementpulver ist bevorzugt in einem Innenraum der Kartusche angeordnet.

In einigen Ausführungsformen weist der Behälter eine Monomerflüssigkeit auf. Die Monomerflüssigkeit ist bevorzugt in einem Innenraum des Behälters angeordnet. Die Monomerflüssigkeit kann in einer Glasampulle oder in einem versiegelten Folienbeutel innerhalb des Behälters angeordnet sein.

In einigen Ausführungsformen weist der Behälter weiterhin einen für eine Monomerflüssigkeit durchlässigen Filter auf. Der Filter ist bevorzugt angeordnet und eingerichtet, ein Eindringen von Glassplittern aus dem Behälter in die Kartusche zu verhindern. Die Vorrichtung kann zur Aufnahme einer Glasampulle mit Monomerflüssigkeit eingerichtet sein. Die Vorrichtung kann weiterhin zum Aufbrechen einer Glasampulle mit Monomerflüssigkeit eingerichtet sein. Die Glasampulle kann insbesondere innerhalb des Behälters aufnehmbar und aufbrechbar sein. Der Behälter kann hierzu eine Aufnahme für eine Glasampulle aufweisen. Der Behälter kann ein Mittel zum Aufbrechen einer Glasampulle aufweisen. Beispielsweise kann der Behälter einen Vorsprung aufweisen, der zum Abbrechen des Kopfs einer Glasampulle angeordnet und eingerichtet ist.

Der Filter kann eingerichtet sein, Bruchstücke einer Glasampulle in dem Behälter zurückzuhalten. Hierdurch kann vermieden werden, dass Glassplitter in die Kartusche gelangen. Hierdurch kann eine Verunreinigung von Knochenzementteig mit Glassplittern vermieden werden.

In einigen Ausführungsformen weist die Vorrichtung einen Mischstab auf. Der Mischstab ist bevorzugt zum Mischen von Monomerflüssigkeit und PMMA-Knochenzementpulver eingerichtet. Der Mischstab kann Flügel aufweisen. Die Flügel können eine homogene Mischung von Monomerflüssigkeit und PMMA-Knochenzementpulver erleichtern. Die Flügel sind bevorzugt an einem Ende des Mischstabs angeordnet, welches innerhalb der Kartusche angeordnet ist. Der Mischstab kann einen, zwei oder mehr als zwei Flügel aufweisen. Der Mischstab kann an einem Ende des Mischstabs, welches bevorzugt außerhalb der Kartusche angeordnet ist, einen Griff aufweisen. Der Mischstab ist bevorzugt beweglich in der Kartusche angeordnet.

Der Mischstab kann ein Austragsrohr aufweisen. Hierbei kann das Austragsrohr zur Abgabe eines in der Vorrichtung gemischten Knochenzementteigs (d.h. eines Knochenzementteigs, der aus Monomerflüssigkeit und PMMA-Knochenzementpulver gemischt wurde) ausgebildet und eingerichtet sein. Bevorzugt ist das Austragsrohr verschließbar. Hierdurch kann verhindert werden, dass Knochenzementteig während des Mischens unerwünscht aus der Kartusche austritt. Das Austragsrohr kann als Hohlraum innerhalb des Mischstabs ausgebildet sein. In einigen Ausführungsformen ist das Austragsrohr durch einen lösbaren Kern verschließbar. Demnach kann der Mischstab einen lösbaren, stabförmigen Kern aufweisen, beispielsweise einen Metallkern. Der Kern kann auch die Festigkeit des Mischstabs verbessern.

Der Mischstab kann eine Sollbruchstelle aufweisen, um den Mischstab nach dem Mischvorgang abzubrechen. Die Sollbruchstelle ist bevorzugt außerhalb der Kartusche angeordnet, so dass der Mischstab ohne ein Öffnen der Kartusche von außen abgebrochen werden kann.

In einigen Ausführungsformen weist der Behälter weiterhin einen Träger zur Aufnahme einer Ampulle auf. Solche Ampullen können insbesondere Glasampullen sein, in denen Monomerflüssigkeit kommerziell erhältlich ist. Der Träger ist bevorzugt angeordnet und eingerichtet, das Aufbrechen einer Ampulle und das Abfließen von Monomerflüssigkeit aus der Ampulle durch den Auslass und das Einleitungsrohr durch freien Gravitationsfluss zu erlauben.

In einigen Ausführungsformen weist der Behälter weiterhin ein Mittel zur Öffnung eines Folienbeutels oder einer Glasampulle innerhalb der Vorrichtung auf. Ein Beispiel für ein Mittel zur Öffnung eines Folienbeutels ist ein Dorn, welcher zum Durchstechen eines Folienbeutels geeignet ist. Ein solcher Dorn kann beispielsweise aus einem starren Kunststoff oder aus Metall ausgebildet sein. Ein Beispiel für ein Mittel zur Öffnung einer Glasampulle ist ein Vorsprung, gegen den der Kopf einer Glasampulle gedrückt werden kann, um sie aufzubrechen. Ein Mittel zur Öffnung einer Glasampulle kann ein flexibles Gehäuseteil aufweisen, so dass die Glasampulle von außen mit der Hand gebrochen werden kann, indem das flexible Gehäuseteil verformt wird. Ein Mittel zur Öffnung einer Glasampulle kann auch ein beweglich angeordnetes Element wie z.B. einen Kolben, aufweisen, welches die Glasampulle gegen ein hartes Gehäuseteil, z.B. einen Vorsprung, drücken und dadurch aufbrechen kann. Mittel zur Öffnung eines Folienbeutels oder einer Glasampulle sind weiterhin in DE19532015A1, WO9718031A1, EP2404864B1 und WO2010012114A1 offenbart.

In einer Ausführungsform weist der Behälter einen in dem Behälter angeordneten Vorsprung auf, der zum Aufbrechen einer in dem Behälter aufnehmbaren Glasampulle ausgebildet und eingerichtet ist, und weist weiterhin einen in dem Behälter beweglich angeordneten Träger zur Aufnahme einer Glasampulle auf, wobei der Träger ausgebildet und eingerichtet ist, eine in der Halterung aufnehmbare Glasampulle durch Druck der Glasampulle gegen den Vorsprung aufzubrechen. Der Träger kann beispielsweise als beweglicher Kolben ausgestaltet sein.

In einer Ausführungsform weist der Behälter einen flexiblen Bereich und einen starren Bereich auf, wobei der flexible Bereich zur Bewegung einer in dem Behälter aufgenommenen Glasampulle gegen den starren Bereich eingerichtet ist, um die Glasampulle aufzubrechen.

Weiterhin kann das Basisteil eine Belüftungsöffnung aufweisen. Die Belüftungsöffnung kann in dem Behälter angeordnet sein. Die Belüftungsöffnung kann bevorzugt oberhalb des Sammelbereichs in dem Basisteil angeordnet sein. Vorzugsweise ist die Belüftungsöffnung derart in dem Basisteil angeordnet, dass ein Kontakt mit Monomerflüssigkeit im Sammelbereich vermieden wird. Die Belüftungsöffnung kann wahlweise als einfache Öffnung oder als Ventil ausgestaltet sein. Die Öffnung kann an der Innenseite durch ein gasdurchlässiges poröses Material abgedeckt sein, so dass das Eindringen von Partikeln von außen in den Behälter vermieden werden kann, und gleichzeitig ein Luftaustausch durch das poröse Material möglich ist. Das Ventil kann beispielsweise ausgestaltet sein, Luft nur in Richtung des Innenraums des Behälters passieren zu lassen. Hierdurch kann der Transfer der Monomerflüssigkeit in die Kartusche verbessert werden, insbesondere wenn der Transfer durch einen Druckunterschied erfolgt. Weiterhin kann ein unidirektionales Ventil das unerwünschte Austreten von Monomerflüssigkeit aus der Vorrichtung verhindern.

Ein weiterer Aspekt betrifft ein Verfahren zur Herstellung eines Knochenzementteigs, wobei das Verfahren die nachfolgenden Schritte aufweist,
Bereitstellen einer Vorrichtung nach einer der hierin beschriebenen Ausführungsformen;
gegebenenfalls Einbringen einer Monomerflüssigkeit in den Behälter;
gegebenenfalls Einbringen eines PMMA-Knochenzementpulvers in die Kartusche;
gegebenenfalls Freisetzen der Monomerflüssigkeit in dem Behälter aus einer Verpackung;
Anordnen der Vorrichtung, so dass die Monomerflüssigkeit aus dem Behälter durch freien Gravitationsfluss in den Sammelbereich überführt wird;
Inkontaktbringen der Kartusche mit dem Basisteil;
Einstechen des Hohldorns durch das Septum, sodass eine fluidleitende Verbindung zwischen dem Sammelbereich und der Kartusche gebildet wird;
Überführen der Monomerflüssigkeit aus dem Behälter in die Kartusche;
Mischen der Monomerflüssigkeit mit dem PMMA-Knochenzementpulver in der Kartusche und dadurch Erhalten eines Knochenzementteigs.

Ein weiterer Aspekt betrifft ein Verfahren zur Herstellung eines Knochenzementteigs, wobei das Verfahren die nachfolgenden Schritte aufweist,
Bereitstellen einer Vorrichtung, welche ein Basisteil mit Monomerflüssigkeit, einem beweglichen Kolben mit einer Durchführung, und einem Hohldorn, und eine Kartusche mit einem Septum und einem PMMA-Knochenzementpulver aufweist;
Drücken der Kartusche gegen den Kolben, sodass die Spitze des Hohldorns aus der Durchführung des Kolbens freigegeben wird;
Einstechen des Hohldorns durch das Septum, sodass eine fluidleitende Verbindung zwischen dem Sammelbereich und der Kartusche gebildet wird;
Überführen der Monomerflüssigkeit aus dem Behälter in die Kartusche;
Mischen der Monomerflüssigkeit mit dem PMMA-Knochenzementpulver in der Kartusche und dadurch Erhalten eines Knochenzementteigs.

Ein weiterer Aspekt betrifft die Verwendung einer hierin beschriebenen Vorrichtung oder des vorangehend beschriebenen Verfahrens zur Herstellung von Knochenzementteig, insbesondere PMMA-Knochenzementteig. Hierbei kann eine Monomerflüssigkeit mit einem PMMA-Knochenzementpulver innerhalb der Vorrichtung gemischt werden, um einen Knochenzementteig zu bilden. Ein solcher Knochenzementteig kann aus der Vorrichtung abgegeben werden und an einem Zielort zu PMMA-Knochenzement aushärten.

### BEISPIELE

Die Erfindung wird nachfolgend anhand von Beispielen weiter verdeutlicht, die jedoch nicht als einschränkend zu verstehen sind. Dem Fachmann wird ersichtlich sein, dass anstelle der hier beschriebenen Merkmale andere äquivalente Mittel in ähnlicher Weise verwendet werden können.

### FIGUREN

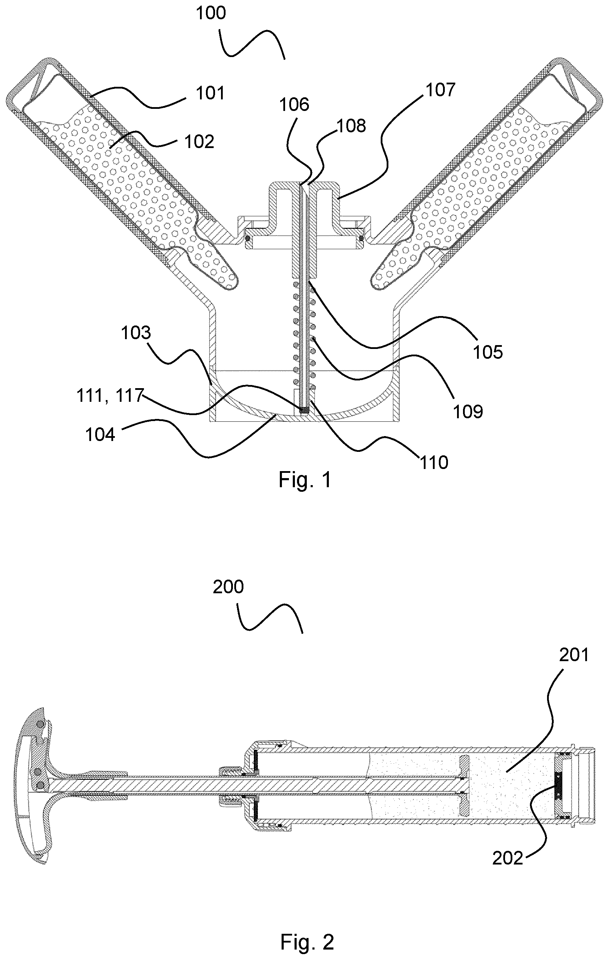

**Figur 1** zeigt beispielhaft eine Ausführungsform eines Basisteils **100** einer erfindungsgemäßen Vorrichtung. Das Basisteil **100** weist einen Behälter **101** auf, der zur Aufnahme einer Monomerflüssigkeit **102** eingerichtet ist. Der Behälter **101** weist weiterhin ein Bodenteil **103** auf, das zum Aufstellen des Basisteils **100** auf eine Unterlage ausgebildet und eingerichtet ist. Hierzu kann das Bodenteil eine ebene Außenfläche und/oder ein oder mehrere Fußelemente aufweisen. Innerhalb des Bodenteils **103** ist ein Sammelbereich **104** angeordnet. Der Sammelbereich **104** ist zur Aufnahme einer Monomerflüssigkeit eingerichtet, die aus dem Behälter **101** abgegeben werden kann. Innerhalb des Basisteils **100** ist ein Hohldorn **105** angeordnet, der sich von dem Sammelbereich **104** zu einer dem Sammelbereich gegenüberliegenden Seite erstreckt. In dem Basisteil 100 ist weiterhin ein Kolben 107 angeordnet, welcher beweglich auf einer Feder 109 gelagert ist. Der Kolben weist eine Durchführung 108 auf, welche als zylinderförmiger Hohlraum ausgestaltet ist. Die Durchführung ist zur Aufnahme des Hohldorns 105 ausgebildet und eingerichtet. Der Hohldorn weist eine Spitze 106 auf, welche in einem Grundzustand des Basisteils in dem Hohlraum aufgenommen ist. Der Hohldorn 105 ist an einem Ende des Hohldorns, welches der Spitze 106 gegenüber liegt, in einer Halterung 110 gelagert, welche im Bodenteil 103 angeordnet ist. Die Halterung 110 weist ein Leitungselement 111 auf, welches hier als Durchbruch durch die Halterung 110 ausgestaltet ist. Das Leitungselement 111 stellt eine fluidleitende Verbindung zwischen dem Sammelbereich 104 und dem Hohldorn 105 her. An dem Leitungselement 111 ist ein Filter 117 angeordnet, welcher das Eindringen von Glasbruchstücken aus dem Sammelbereich 104 in den Hohldorn 105 verhindern kann.
Figur 2 zeigt eine Kartusche 200 einer erfindungsgemäßen Vorrichtung. Die Kartusche 200 ist zur Aufnahme eines PMMA-Knochenzementpulvers 201 ausgebildet und eingerichtet. Die Kartusche 200 weist ein Septum 202 auf, welche die Kartusche nach außen begrenzt. Das Septum 202 ist flüssigkeitsdicht mit dem Gehäuse der Kartusche verbunden. Die Kartusche ist ausgebildet und eingerichtet, mit dem Basisteil 100 in Kontakt gebracht zu werden, um eine Monomerflüssigkeit aus dem Basisteil 100 in die Kartusche aufzunehmen.

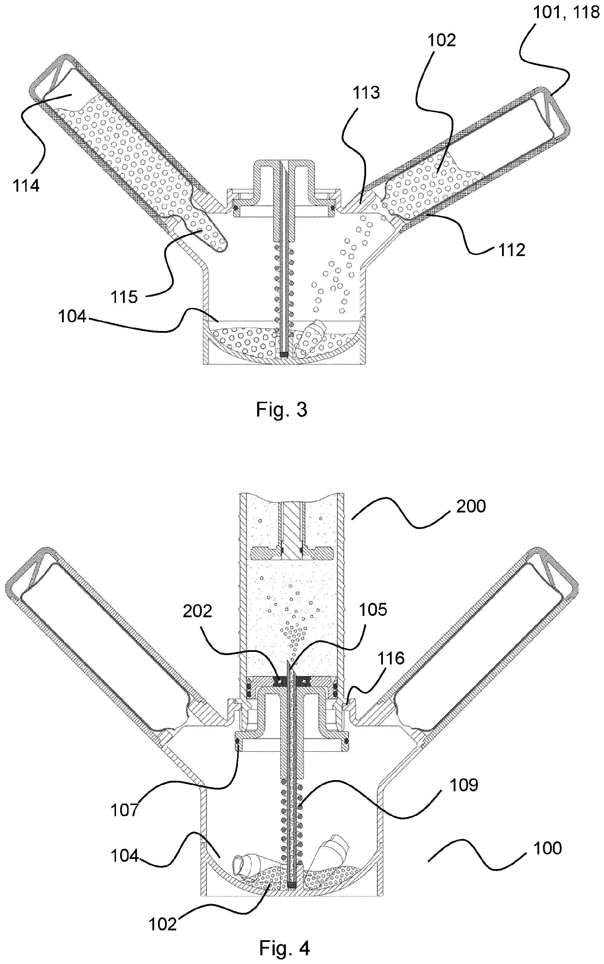

Figur 3 zeigt ein Basisteil 100 mit einem Behälter 101, welcher einen Träger 118 zur Aufnahme einer Glasampulle 114 aufweist. Der Behälter 101 weist einen flexiblen Bereich 112 und einen starren Bereich 113 auf. Ein Benutzer kann den flexiblen Bereich 112 biegen, um die in dem Behälter 101 aufgenommene Glasampulle 114 mit dem Ampullenkopf 115 gegen den starren Bereich 113 zu drücken, um den Ampullenkopf 115 abzubrechen und dadurch die Monomerflüssigkeit 102 aus der Glasampulle 114 freizusetzen. Die Monomerflüssigkeit 102 kann durch freien Gravitationsfluss in den Sammelbereich 104 abfließen.
Figur 4 zeigt ein Basisteil 100, welches mit einer Kartusche 200 in Kontakt gebracht ist. Der Hohldorn 105 ist durch das Septum 202 geführt, um eine fluidleitende Verbindung zwischen dem Sammelbereich 104 und dem Innenraum der Kartusche 200 auszubilden. Somit kann Monomerflüssigkeit 102 aus dem Sammelbereich 104 in die Kartusche 200 überführt werden, beispielsweise durch Anschluss einer Vakuumquelle an die Kartusche 200. Das Basisteil 100 weist einen Anschlag 116 auf, der eingerichtet ist, in einen Vorsprung an der Kartusche 200 einzugreifen. Durch Druck der Kartusche 200 gegen den Kolben 107 wird der Kolben in das Innere des Basisteils **100** bewegt, wobei diese Bewegung durch den Anschlag **116** begrenzt ist. Der Anschlag **116** kann weiterhin die Bewegung des Kolbens **107** nach außen begrenzen, um zu verhindern, dass die Feder **109** den Kolben **107** vollständig aus dem Basisteil **100** heraus bewegt.

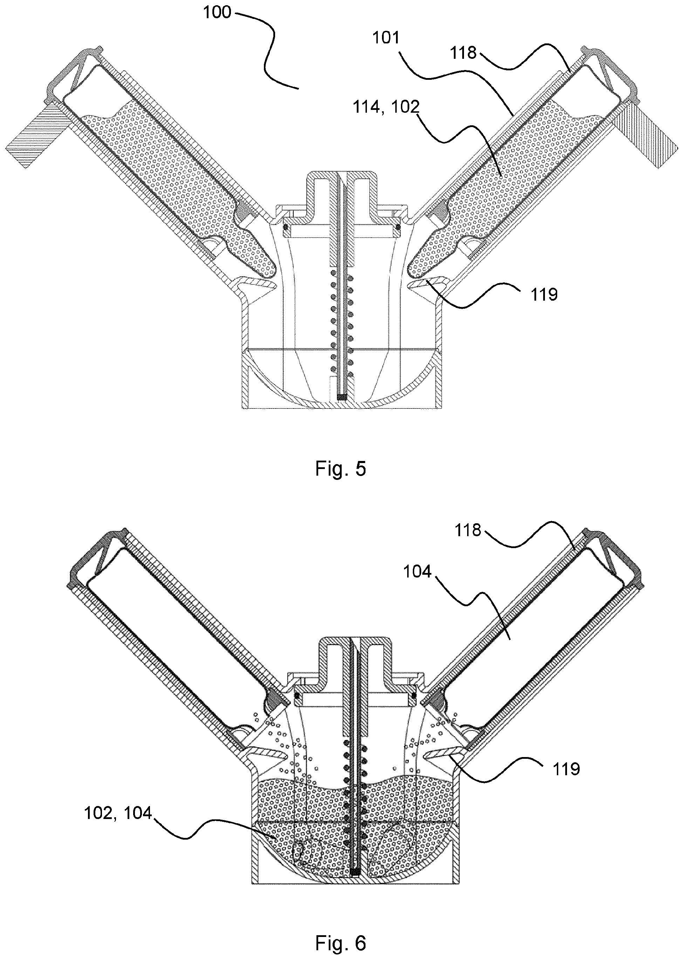

**Figur 5** zeigt ein Basisteil **100** mit einem Behälter **101,** welcher einen Träger **118** zur Aufnahme einer Glasampulle **114** aufweist. Der Träger **118** ist beweglich in dem Behälter **101** angeordnet. Der Behälter **101** weist weiterhin einen Vorsprung **119** auf. Mithilfe des beweglichen Trägers **118** kann eine Glasampulle **114** gegen den Vorsprung **119** gedrückt werden, um die Glasampulle aufzubrechen und eine Monomerflüssigkeit **102** aus der Glasampulle freizusetzen.
**Figur 6** zeigt ein Basisteil **100** gemäß Figur 5, wobei eine Glasampulle **114** durch Druck des beweglichen Trägers **118** gegen den Vorsprung **119** aufgebrochen ist, um eine Monomerflüssigkeit **102** in den Sammelbereich **104** abzugeben.

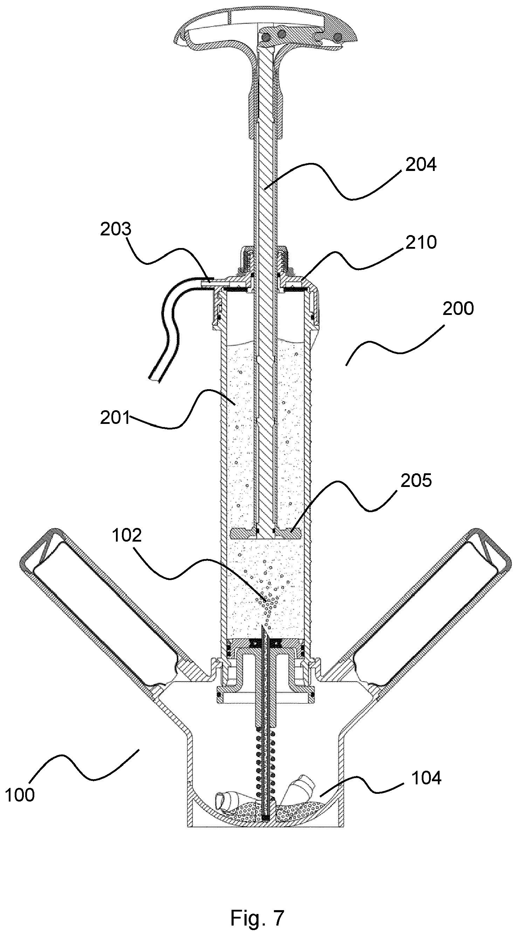

**Figur 7** zeigt eine erfindungsgemäße Vorrichtung, wobei die Kartusche **200** einen Anschluss **203** für eine Vakuumquelle aufweist. Hiermit kann ein Unterdruck in der Kartusche **200** erzeugt werden, um eine Monomerflüssigkeit **102** aus dem Sammelbereich **104** des Basisteils **100** in die Kartusche **200** überführen. Die Kartusche enthält ein PMMA-Knochenzementpulver **201,** welches mit der Monomerflüssigkeit **102** zu einem Knochenzementteig gemischt werden kann. Hierzu weist die Kartusche **200** weiterhin einen Mischstab **204** mit einer Mischscheibe **205** auf. Der Mischstab **204** ist durch einen auf die Kartusche **200** aufgeschraubten Kartuschenkopf **210** geführt. Der Anschluss **203** ist an dem Kartuschenkopf **210** angeordnet. Die Mischscheibe **205** ist an einem Ende des Mischstabs **204** angeordnet, welches im Innenraum der Kartusche **200** angeordnet ist.

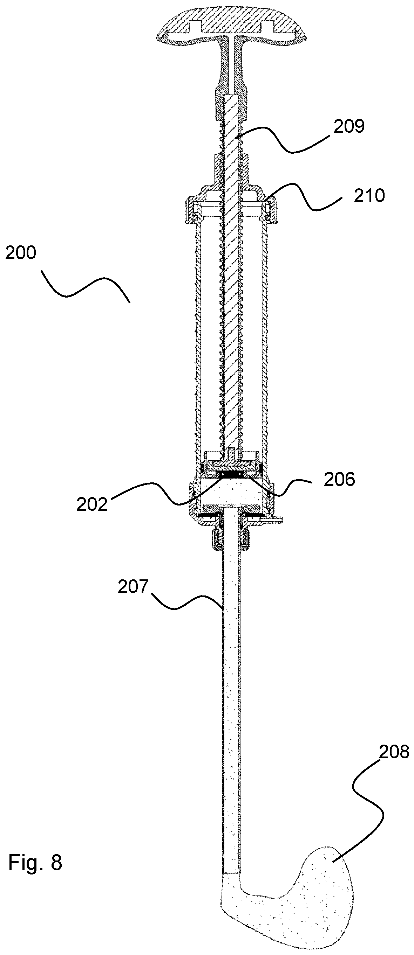

**Figur 8** zeigt eine Kartusche **200,** welche eine Gewindestange **209** aufweist. Die Gewindestange **209** greift in einen Kartuschenkopf **210** ein, um die Gewindestange **209** in die Kartusche einschrauben zu können. Hierdurch kann eine Halterung **206** für das Septum **202** innerhalb der Kartusche **200** wie ein Kolben bewegt werden, um in der Kartusche angemischten Knochenzementteig **208** aus der Kartusche **200** durch ein Austragsrohr **207** abzugeben.

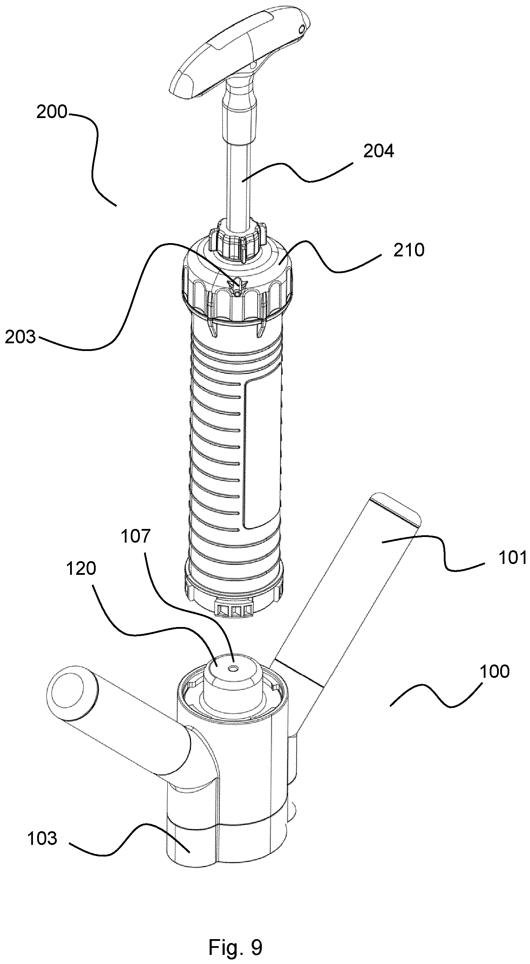

**Figur 9** zeigt eine Ausführungsform einer erfindungsgemäßen Vorrichtung, welche ein Basisteil **100** und eine damit verbindbare Kartusche **200** aufweist. Das Basisteil **100** weist ein Schraubgewinde zur Verbindung mit der Kartusche **200** auf. Das Basisteil **100** weist weiterhin einen nach außen hervorstehenden Kontaktbereich **120** auf, der in die Kartusche **200** aufnehmbar ist. Ein Kolben **107** und ein Hohldorn **105** sind an diesem Kontaktbereich **120** angeordnet. Das Basisteil **100** weist weiterhin ein Bodenteil **103** auf, welches während der Verwendung der Vorrichtung auf eine Unterlage aufgestellt werden kann. Das Basisteil **100** weist weiterhin einen Behälter **101** zur Aufnahme von Glasampullen mit Monomerflüssigkeit auf. Der Behälter **101** weist eine verschließbare Öffnung zur Aufnahme einer Glasampulle auf. Die Kartusche **200** weist einen Kartuschenkopf **210** mit einem Vakuumanschluss **203** auf. Die Kartusche **200** weist weiterhin einen Mischstab **204** auf, der durch den Kartuschenkopf **210** in den Innenraum der Kartusche **200** geführt ist.

### BEZUGSZEICHENLISTE

- 100: Basisteil
- 101: Behälter
- 102: Monomerflüssigkeit
- 103: Bodenteil
- 104: Sammelbereich
- 105: Hohldorn
- 106: Spitze
- 107: Kolben
- 108: Durchführung
- 109: Feder
- 110: Halterung für Hohldorn
- 111: Leitungselement
- 112: flexibler Bereich des Behälters
- 113: starrer Bereich des Behälters
- 114: Glasampulle
- 115: Ampullenkopf
- 116: Anschlag
- 117: Filter
- 118: Träger
- 119: Vorsprung
- 120: Kontaktbereich
- 200: Kartusche
- 201: PMMA-Knochenzementpulvers
- 202: Septum
- 203: Anschluss für Vakuumquelle
- 204: Mischstab
- 205: Mischscheibe
- 206: Halterung für Septum
- 207: Austragsrohr
- 208: Knochenzementteig
- 209: Gewindestange
- 210: Kartuschenkopf

## Patentansprüche

1. Vorrichtung zur Herstellung von Knochenzement, aufweisend
ein Basisteil (100), welches einen Behälter (101) zur Aufnahme einer Monomerflüssigkeit (102), ein Bodenteil (103) mit einem Sammelbereich (104), und einen Hohldorn (105) mit einer Spitze (106) aufweist,
und eine Kartusche (200) zur Aufnahme eines PMMA-Knochenzementpulvers (201), wobei die Kartusche ein Septum (202) aufweist, welches die Kartusche nach außen begrenzt,
wobei die Vorrichtung ausgebildet und eingerichtet ist, mittels Durchstechen des Septums mit dem Hohldorn eine fluidleitende Verbindung zwischen dem Basisteil und der Kartusche zu bilden, um eine Monomerflüssigkeit aus dem Sammelbereich in die Kartusche zu überführen.

2. Vorrichtung gemäß Anspruch 1, wobei das Basisteil weiterhin einen Kolben (107) aufweist, wobei der Kolben eine Durchführung (108) zur Aufnahme des Hohldorns aufweist, wobei der Kolben eingerichtet ist, in einer Grundkonfiguration der Vorrichtung die Spitze des Hohldorns in der Durchführung aufzunehmen, und die Spitze des Hohldorns aus der Durchführung freizugeben, wenn die Kartusche auf den Kolben gedrückt wird.

3. Vorrichtung gemäß Anspruch 2, wobei das Basisteil eine Feder (109) aufweist, welche zur beweglichen Lagerung des Kolbens innerhalb des Basisteils angeordnet und eingerichtet ist.

4. Vorrichtung gemäß einem der vorangehenden Ansprüche 2 oder 3, wobei das Basisteil einen Anschlag (116) aufweist, um die Bewegung des Kolbens und/oder der Kartusche in dem Basisteil zu begrenzen.

5. Vorrichtung gemäß einem der vorangehenden Ansprüche, wobei die Vorrichtung einen Filter aufweist, wobei der Filter (117) angeordnet und ausgebildet ist, das Eindringen von Partikeln aus dem Basisteil in die Kartusche zu verhindern.

6. Vorrichtung gemäß einem der vorangehenden Ansprüche, wobei die Kartusche einen Anschluss (203) zur Verbindung mit einer Vakuumquelle aufweist, um das Überführen einer Monomerflüssigkeit aus dem Sammelbereich in die Kartusche mithilfe eines Druckunterschieds zu ermöglichen.

7. Vorrichtung gemäß einem der vorangehenden Ansprüche, wobei der Behälter einen Träger (118) zur Aufnahme einer Glasampulle oder eines Folienbeutels mit Monomerflüssigkeit aufweist.

8. Vorrichtung gemäß einem der vorangehenden Ansprüche, wobei der Behälter einen Ampullenbrecher (113) aufweist, wobei der Ampullenbrecher zum Öffnen einer Glasampulle innerhalb des Behälters eingerichtet ist.

9. Vorrichtung gemäß Anspruch 8, wobei der Ampullenbrecher ausgestaltet und eingerichtet ist, durch Biegen des Behälters relativ zu dem Bodenteil, oder durch Verschieben eines Trägers (118) gegen einen Vorsprung (119), eine Glasampulle innerhalb des Behälters zu öffnen.

10. Vorrichtung gemäß einem der vorangehenden Ansprüche, wobei das Basisteil eingerichtet ist, das Überführen einer Monomerflüssigkeit aus dem Behälter in den Sammelbereich durch freien Gravitationsfluss zu ermöglichen.

11. Vorrichtung gemäß einem der vorangehenden Ansprüche, wobei der Behälter in einem Winkel zu dem Bodenteil angeordnet ist, welcher 20° bis 80° beträgt.

12. Vorrichtung gemäß einem der vorangehenden Ansprüche, wobei die Kartusche lösbar mit dem Basisteil verbindbar ist.

13. Vorrichtung gemäß einem der vorangehenden Ansprüche, wobei das Basisteil ein Leitungselement (111) aufweist, wobei das Leitungselement eingerichtet ist, eine fluidleitende Verbindung zwischen dem Sammelbereich und dem Hohldorn herzustellen.

14. Vorrichtung gemäß Anspruch 13, wobei der Hohldorn in einer zylinderförmigen Halterung (110) gelagert ist, wobei die Halterung in dem Sammelbereich angeordnet ist.

15. Vorrichtung gemäß einem der vorangehenden Ansprüche, wobei der Sammelbereich eine Vertiefung aufweist, und wobei der Hohldorn bevorzugt fluidleitend mit dem tiefsten Punkt der Vertiefung verbunden ist.

16. Vorrichtung gemäß einem der vorangehenden Ansprüche, wobei die Vorrichtung ausgestaltet und eingerichtet ist, eine Monomerflüssigkeit aus dem Basisteil in die Kartusche zu überführen, während das Basisteil auf einer Unterlage aufgestellt ist, so dass das Bodenteil in Richtung der Unterlage ausgerichtet ist.
